(19) **Europäisches Patentamt**
**European Patent Office**
**Office européen des brevets**

(11) **EP 2 979 605 A1**

(12) **EUROPEAN PATENT APPLICATION**
published in accordance with Art. 153(4) EPC

(43) Date of publication:
**03.02.2016 Bulletin 2016/05**

(51) Int Cl.:
**A61B 1/00** (2006.01)

(21) Application number: **13879730.3**

(22) Date of filing: **29.03.2013**

(86) International application number:
**PCT/JP2013/059725**

(87) International publication number:
**WO 2014/155725 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Tokyo Institute of Technology Tokyo 152-8550 (JP)**

(72) Inventors:
• **KAWASHIMA Kenji**
  **Tokyo 152-8550 (JP)**
• **TADANO Kotaro**
  **Tokyo 152-8550 (JP)**

(74) Representative: **Reichert & Lindner Partnerschaft Patentanwälte Bismarckplatz 8 93047 Regensburg (DE)**

(54) **ENDOSCOPIC OPERATING SYSTEM AND ENDOSCOPIC OPERATING PROGRAM**

(57) Provided is an endoscopic operating system, including: a sensor section for detecting movement of at least one of a head part and an upper body of an operator; a control section for driving one or more actuators, corresponding to the movement detected by the sensor section; a holding arm unit supported to be reciprocatable and rotatable by the actuator and one or more displacing mechanisms connected to the actuator; an image capturing section provided at an arbitrary part of the holding arm unit through a joint section capable of freely change an image capturing angle by the actuator; and a display section for displaying an image captured by the image capturing section on a screen.

## FIG. 1

## Description

Technical Field

The present invention relates to an endoscopic operating system and an endoscopic operating program.

Background Art

In a field of surgery, endoscopic surgery is widely performed instead of abdominal surgery because of the advantages of endoscopic surgery such as quick recovery after surgery and the small size of a cut made by surgery. For such endoscopic surgery, a master-slave endoscopic operating system allowing remote control has been proposed. As disclosed for example by Patent Literature 1, on such an endoscopic operating system, the magnification factor of the zoom lens of an endoscope is controlled based on a detection output from an attitude sensor that is arranged in a head mount display (hereinafter, also referred to as an HMD) to detect the movement of the head of an operator. The movement of the head part of the operator is recognized by a displacement of the attitude sensor relative to a magnetic source generating a magnetic field. In such a manner, for example, when the operator turns left with respect to a patient, a left image based on captured image data obtained through the solid-state image sensing device of the endoscope is displayed on a pair of liquid crystal monitors in the HMD, and when the operator moves toward the patient, a visual field magnified by the zoom lens is obtained. Accordingly, the operator can three dimensionally observe the inside of the body cavity into which the endoscope has been inserted.

The endoscope grapping device disclosed in Non-patent Literature 1 is configured with a five node link mechanism, a ball joint section for holding a tracker penetrating through the abdominal wall by the abdominal wall part, and a driving section and an operating section for driving the link mechanism. With this configuration, the laparoscope, which is a kind of endoscopes, is a zoom type, can quickly switch a short distance and a long distance of a screen and a distant screen, and it is considered that the zoom type laparoscope can quickly move by a controller switch to a position that the operator wants.

Related Art Documents

Patent Literature

Patent Literature 1: JP 10-309258 A

Non-Patent Literature

Non-Patent Literature 1 : Iryo-yo Naishikyo Haji Souchi (Medical Endoscope Gripping Device) "Naviot" , catalog, issued by Hitachi Hybrid Network Co., Ltd.

Disclosure of the Invention

Problems to be Solved by the Invention

As shown in FIG. 4, on each device described in Patent Literature 1 and Non-Patent Literature 1, if the image capturing direction of an endoscope is parallel (image capturing angle $\theta=0°$) with the direction of a holding arm unit 110 holding the endoscope (a case of a so-called straight view scope), when at least one of the head part and the upper body of an operator OP is moved forward or backward, an endoscope 124 also moves forward or backward in association. Accordingly, image capturing is performed on an image capturing object as a near image (zoomed in) and as a distant image (zoom out) to be displayed on a display section 132 that displays an image captured by the endoscope 124 on a screen. Thus, it is possible to intuitively operate the endoscope 124 without a particular problem.

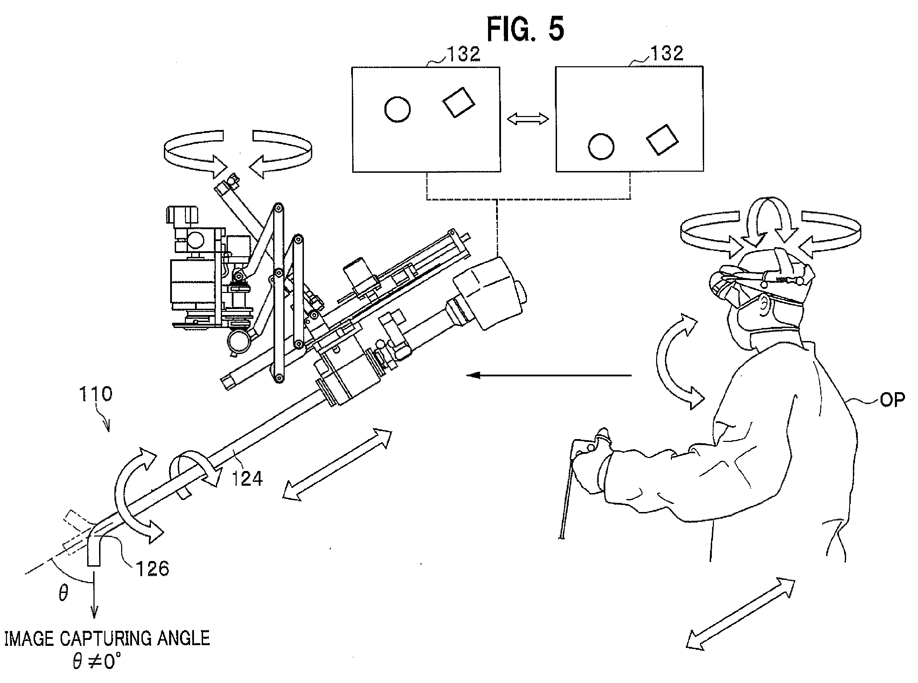

However, as shown in FIG. 5, if the image capturing direction of the endoscope 124 is made different (image capturing angle $\neq 0°$) from the direction of the holding arm unit 110 holding the endoscope 124 (a case of a so-called oblique view scope), there is a problem that intuitive operation is impossible unlike a straight view scope. Concretely, for example, if the endoscope 124 is directed vertically downward by a joint section 126 provided on the holding arm unit 110, when at least one of the head part and the upper body of the operator OP is moved forward or backward likewise as above in order to capture a zoomed-in image or a zoomed-out image, the endoscope 124 that is capturing an image downward is moved forward or backward. Accordingly, on the display section 132, an image captured downward is only moved upward or downward (forward or backward), and a zoomed-in image or a zoomed-out image cannot be obtained.

Incidentally, if it is tried to capture a zoomed-in image or a zoomed-out image while the endoscope 124 is capturing an image downward, the operator OP needs not to move forward or backward at least one of the head part and the upper body of the operator OP, but needs to change the inclination angle of at least one of the head part and the upper body, or to perform bending and stretching exercises in order to move upward or downward the endoscope 124 or perform zooming in or zooming out. Accordingly, there is a problem with an oblique view scope of impossibility of intuitive operation as described above unlike a straight view scope.

An object of the present invention is to provide an endoscopic operating system and an endoscopic operating program enabling intuitive operation regardless of the image capturing angle of an endoscope.

Means for Solving the Problems

[1] An endoscopic operating system includes: a sensor section for detecting movement of at least one of a head part and an upper body of an operator; a control section for driving one or more actuators, corresponding to the movement detected by the sensor section; a holding arm unit supported to be reciprocatable and rotatable by the actuator and one or more displacing mechanisms connected to the actuator; an image capturing section provided at an arbitrary part of the holding arm unit through a joint section capable of freely change an image capturing angle by the actuator; and a display section for displaying an image captured by the image capturing section on a screen, wherein the control section includes: a computing unit for computing an angular velocity and a translation velocity from the movement detected by the sensor section; a transforming unit for transforming the angular velocity and the translation velocity into a target angular velocity vector and a target translation velocity vector of the holding arm unit, taking into account the image capturing angle of the image capturing section by the joint section, and further performing transformation into a velocity target value of the displacing mechanism by using the target angular velocity vector and the target translation velocity vector in order to obtain a position target value from the velocity target value; and a drive control unit for driving the actuator according to the position target value.

The endoscopic operating system according to the invention is arranged as follows. The control section computes the movement of the operator, i.e., the angular velocity and the translation velocity of at least one of the head part and the upper body, taking into account the image capturing angle of the image capturing section by the joint section; transforms these into the target angular velocity vector and the target translation velocity vector of the holding arm unit; further transforms into the velocity target value of the displacing mechanism, using these, to obtain the position target value from this velocity target value; and drives the actuator, according to this position target value. Thus, intuitive operation is possible, regardless of the image capturing angle of the endoscope.

[2] The endoscopic operating system according to the invention is preferably arranged such that: spatial coordinates of the sensor section for detecting the angular velocity and the translation velocity of the head part of the operator are spatial coordinates with a central axis of the neck of the operator as y axis, leftward-rightward direction of the operator as x axis, and forward-backward direction of the operator as z axis; special coordinates of the image capturing section are spatial coordinates with leftward-rightward direction of the image capturing section as x axis, upward-downward direction of the image capturing section as y axis, and optical axis direction of the image capturing section as z axis; and control is performed to make variation of position and acceleration of the head part of the operator and corresponding position variation of the image capturing section are the same, regardless of a bending state of the holding arm unit and the joint section.

In the endoscopic operating system according to the invention, the spatial coordinates of the operator and the spatial coordinates of the image capturing section agree with each other, and the position variation of the image capturing section agrees, corresponding to the variation of the position and the acceleration of the head part of the operator. Accordingly, intuitive operation is possible, regardless of the image capturing angle of the endoscope.

[3] The endoscopic operating system according to the invention is preferably arranged such that: in performing the control, the image capturing angle of the image capturing section is represented by a matrix, and the matrix is used in coordinate transformation from the variation of the head part of the operator into position variation of the holding arm unit and the joint section.

In the endoscopic operating system according to the present invention, the image capturing angle of the image capturing section is represented by a matrix, and this is used for coordinate transformation from the action of the head part of the operator to the action of the holding arm unit. Accordingly, the spatial coordinates of the operator and the spatial coordinates of the image capturing section agree with each other more surely, and the position variation of the image capturing section correspondingly agrees with the variation of the position and the acceleration of the head part of the operator. Accordingly, intuitive operation is possible, regardless of the image capturing angle of the endoscope.

[4] The endoscopic operating system according to the present invention is preferably arranged such that the transformation unit transforms the angular velocity and the translation velocity into the target angular velocity vector and the target translation velocity vector of the holding arm unit, based on following Expressions (1) and (2).

$$\omega_{ref} = R_h\, R_e\, T \cdot \omega'_{cmd} \ \dots(1)$$

$$v_{ref} = R_h\, R_c\, T \cdot v'_{cmd} \ \dots(2)$$

In Expressions (1) and (2),

$\omega_{ref}$ represents a target angular velocity vector of the holding arm unit,
$v_{ref}$ represents a target translation velocity vector of the holding arm unit,
and $R_h$ represents a matrix representing attitude of the holding arm unit and is obtained by computation of forward kinematics of Expression (3) below from displacement by the displacing mechanism,
$R_c$ represents a matrix representing image capturing angle $\theta$ of the image capturing section and expressed by Expression (4) below,
T represents a transformation matrix for transformation from a coordinate system that is set for the sensor section into a coordinate system that is set for the holding arm unit,

$\omega'_{cmd}$ is obtained by limiting an angular velocity instruction vector $\omega_{cmd}$ of the holding arm unit by a limiting value, the angular velocity instruction vector $\omega_{cmd}$ being expressed by Expression (5) below,

and $v'_{cmd}$ is obtained by limiting a translation velocity instruction vector $v_{cmd}$ of the holding arm unit by a limiting value, the translation velocity instruction vector $v_{cmd}$ being expressed by Expression (6) below.

$$R_h = E^{iq1}\, E^{jq2}\, E^{kq4} \dots (3)$$

$$R_c = E^{j\theta} \dots (4)$$

$$\omega_{cmd} = K_r \cdot \omega_s \dots (5)$$

$$v_{cmd} = (0,\, 0,\, K_z\, v_z)^{\,t} \dots (6)$$

In Expressions (3) to (6),

E represents a rotation matrix,
i, j, and k respectively represent rotations around x, y, and z axes
q1, q2, and q4 represent respective displacements by the displacing mechanism,
$\theta$ represents the image capturing angle of the image capturing section,
$K_r$ represents a factor matrix representing a velocity gain,
$\omega_s$ represents a three dimensional angular velocity vector detected by the sensor section,
$K_z$ represents a gain that is set by a user,
$v_z$ represents a velocity in head part forward-backward direction,
and t represents that the matrix is a transposed matrix.

In the endoscopic operating system according to the present invention, the transforming unit transforms the angular velocity and the translation velocity computed from the action of at least one of the head part and the upper body into the target angular velocity vector and the target translation velocity vector of the holding arm unit by Expressions (1) and (2), introducing the matrix Rc expressed by Expression (4) in order to take into account the image capturing angle of the image capturing section by the joint section, and thereafter the actuator is driven. Accordingly, intuitive operation can be more surely performed, regardless of the image capturing angle of the endoscope.

[5] An endoscopic operating program according to the present invention is am endoscopic operating program for operating the endoscopic operating system according to above [1], wherein the program makes a computer function as: a computing unit for computing an angular velocity and a translation velocity from a movement detected by the sensor section; a transforming unit for transforming the angular velocity and the translation velocity into a target angular velocity vector and a target translation velocity vector of the holding arm unit, taking into account image capturing angle of the image capturing section by the joint section, and further performing transformation, by use of these, into a velocity target value of the displacing mechanism to thereby obtain a position target value from the velocity target value; and a drive control unit for driving the actuator, according to the position target value.

The endoscopic operating program according to the invention can make a computer function as the above-described computing unit, the transforming unit, and the drive control unit. Accordingly, intuitive operation is possible, regardless of the image capturing angle of the endoscope.

Advantages of the Invention

By an endoscopic operating system according to the present invention, the action of an operator is transformed into a target angular velocity vector and a target translation velocity vector of the holding arm unit, taking into account the image capturing angle of the image capturing section by the joint section; further transforms into a velocity target value of a displacing mechanism by the use of these; obtains the position target value from this velocity target value; and drives the actuator, according to this position target value. Accordingly, intuitive operation is possible, regardless of the image capturing angle of the endoscope.

By an endoscopic operating program according to the present invention, a computer transforms the action of an operator into a target angular velocity vector and a target translation velocity vector of the holding arm unit, taking into account the image capturing angle of the image capturing section by the joint section; further transforms into a velocity target value of a displacing mechanism by the use of these; obtains a position target value from this velocity target value; and drives the actuator, according to this position target value. Accordingly, intuitive operation is possible, regardless of the image capturing angle of the endoscope.

Brief Description of the Drawings

FIG. 1 is an entire configuration diagram showing one embodiment of an endoscopic operating system according to the present invention wherein an operator is also shown;
FIG. 2 is a block diagram showing the configuration in the one embodiment of the endoscopic operating system according to the invention;

FIG. 3 is a block diagram illustrating the process by a computing unit and a transforming unit of a velocity control computing section;

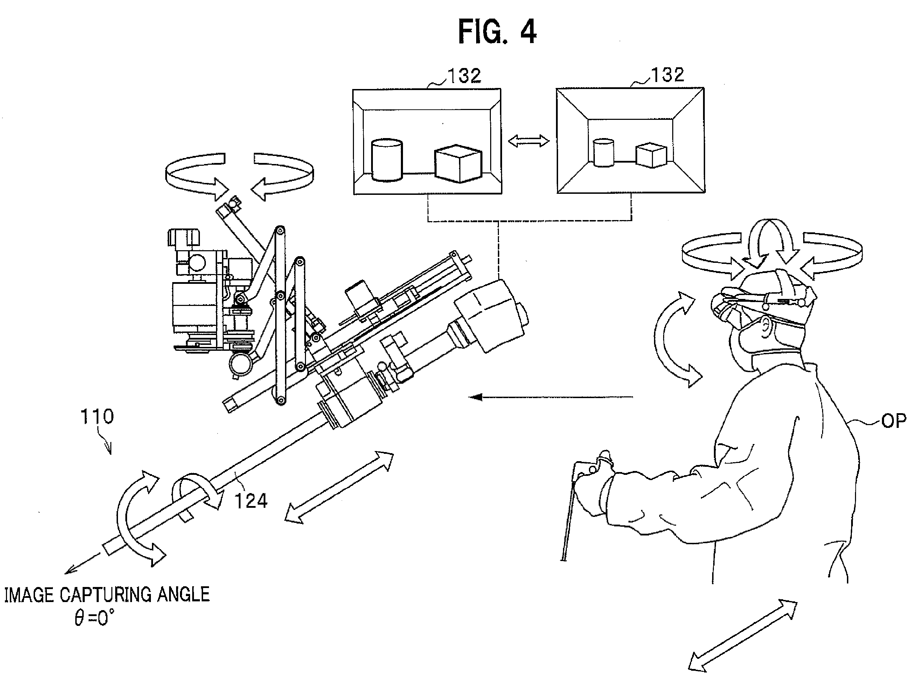

FIG. 4 is a schematic illustration showing an example of an embodiment of a conventional endoscopic operating system; and

FIG. 5 is a schematic illustration showing another example of an embodiment of a conventional endoscopic operating system.

Embodiment for Carrying Out the Invention

In the following, an embodiment of an endoscopic operating system and an endoscopic operating program according to the present invention will be described in detail, referring to the drawings, as appropriate.

[Endoscopic Operating System]

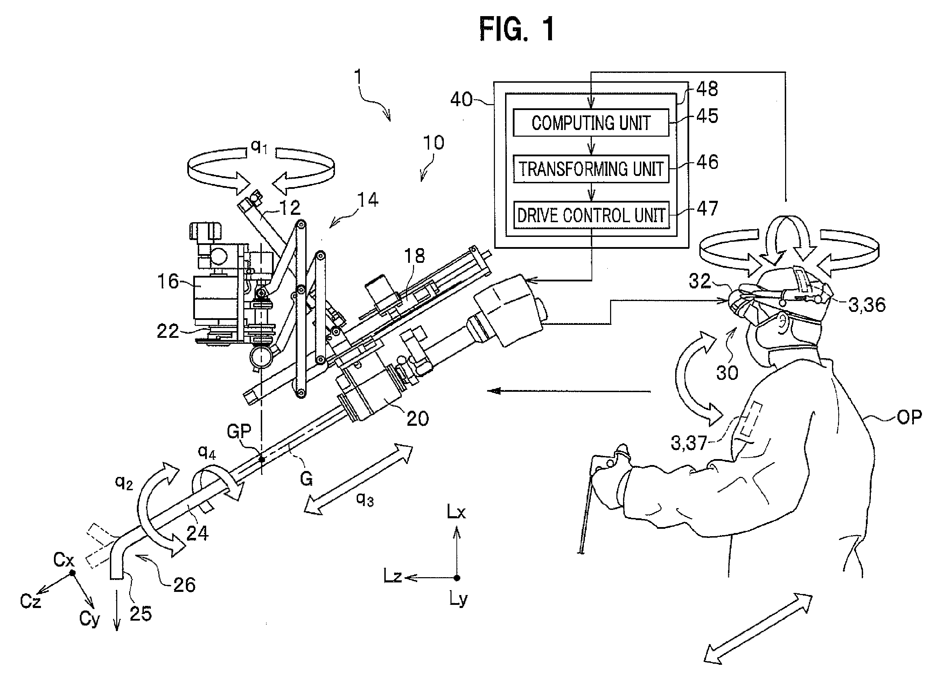

FIG. 1 is an entire configuration diagram showing one example of an endoscopic operating system 1 according to the present invention wherein an operator (surgery operator) OP is also shown.

In FIG. 1, the endoscopic operating system 1 is provided with a sensor section 3, a control section 40 connected with the sensor section 3, a holding arm unit 10 connected with the control section 40, an endoscope 24 held by the holding arm unit 10, and display sections 32 for displaying on a screen an image captured by the endoscope 24.

Incidentally, the endoscope 24 is provided with an image capturing section 25 arranged at an arbitrary part of the holding arm unit 10 through a joint section 26 capable of freely changing the image capturing angle by an actuator. The endoscope 24 is arranged to function as an oblique view scope and a straight view scope by this joint section 26.

The control section 40 includes a computing unit 45, a transforming unit 46, and a drive control unit 47.

The display sections 32 are arranged inside a head mount display 30 (hereinafter, also referred to as an HMD 30) removably attached to the head part of the operator OP.

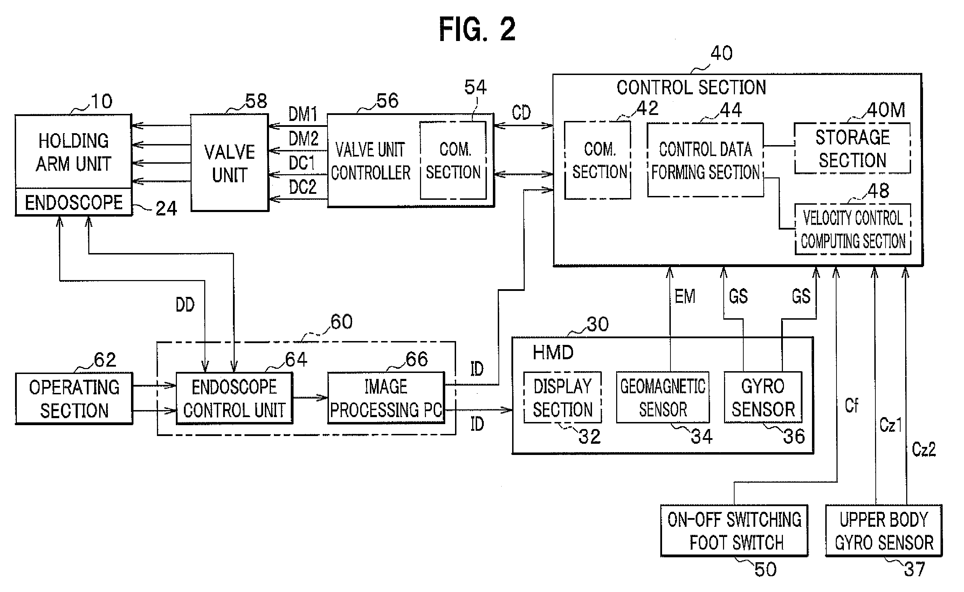

The endoscopic operating system 1 according to the present embodiment shown in FIG. 1 is arranged to perform intuitive translation operation of the visual field such as zooming in and zooming out for an image to be captured by forward and backward translating the head part or the upper body similarly to everyday action. In most cases, as the whole upper body is inclined in moving the head part forward or backward, not only the translation movement of the head part is directly detected, but also the inclination angular velocity of the upper body is also detected by attaching a sensor section 3, for example, a gyro sensor 36 (also called a gyroscope or the like) attached to the head part of the operator OP, an upper body gyro sensor 37 attached to the chest part, and geomagnetic sensors 34 (see FIG. 2). The translation operation of the visual field is realized, using an output value of this detection.

Concretely, for the endoscopic operating system 1, in the spatial coordinate system of the sensor section 3 for detecting the angular velocity and the translation velocity of the head part of the operator OP, the central axis of the neck of the operator OP is defined as y axis, the leftward-rightward direction of the operator OP is defined as x axis, and the forward and backward direction of the operator OP is defined as z axis. In the spatial coordinate system of the image capturing section 25, the leftward-rightward direction of the image capturing section 25 is defined as x axis, the upward-downward direction is defined as y axis, and the optical axis direction is defined as z axis, wherein control is performed such that the variation in the position of the image capturing section 25 and the corresponding variation in the position and the acceleration of the heat part of the operator OP are the same, regardless of the bending state between the holding arm unit 10 and the joint section 26. Incidentally, in order to perform such control, it is preferable that the image capturing angle of the image capturing section 25 is represented by a matrix, and the matrix is used for coordinate transformation from the action of the heat part of the operator OP to the action of the joint section 26 of the holding arm unit 10 and the joint section 26. If such a configuration is adopted for the endoscopic operating system 1, the spatial coordinates of the operator OP and the spatial coordinates of the image capturing section 25 agree with each other, and the position variation of the image capturing section 25 agrees with the variation of the position and the acceleration of the head part of the operator OP so that it is possible to perform intuitive operation regardless of the image capturing angle of the endoscope 24.

For example, as shown in FIG. 1, the sensor section 3 including the gyro sensor 36, the upper body gyro sensor 37, and the geomagnetic sensors 34 is attached to the head part or the chest part of the operator OP to thereby detect the inclination angular velocity of the upper body. Then, from this detected inclination angular velocity of the upper body, the forward-backward translation velocity of the head part is computed to be used as an instruction value for zooming operation and the like. For example, if the upper body is inclined forward, the visual field is zoomed in, and if the upper body is inclined backward, the visual field is zoomed out.

Incidentally, in order to perform easier and more intuitive zooming operation of the visual field of the endoscope 24, when a person naturally moves the heat part with forward-backward and leftward-rightward translation, not only the movement of the neck and a higher part but also the rotation movement with inclination from the upper body, i.e., the velocity of the rotation movement ,with the vicinity of the waist as the center (upper body inclination angular velocity), is preferably detected by the sensor section 3 (the upper body gyro sensor 37). Thus, the forward-backward translation velocity of the head part can be computed from the angular velocity of the upper body, and can be used as an instruction value of the zooming operation and the like. Further, as described later, movement of the head part with five degrees of freedom at least can be detected by combining this and outputs from the sensor section 3, such as the gyro sensor 36, the gyro sensor 37, and the geomagnetic sensors 34.

As described above, the endoscope 24 is provided with the image capturing section 25 arranged at an arbitrary part of the holding arm unit 10 through a joint section capable of freely changing the image capturing angle by an actuator.

Further, the endoscope 24 is configured, including an operating section 62 (see FIG. 2) for performing control of the optical system of the image capturing section 25 and a connecting section (not shown) connected to the operating section 62 to connect a light source and the like to the operating section 62.

The image capturing section 25 is configured, including an optical section (not section) with an objective lens and the like, a solid-state image sensing device (not shown), and a zooming mechanism section (not shown) that includes an actuator (not shown) and controls the lenses of the optical section to magnify or reduce an image obtained by the image capturing section 25. The zooming mechanism section of the image capturing section 25 is controlled by a later-described endoscope control unit (see FIG. 2). A light guide (not shown) is provided adjacent to the objective lens of the image capturing section 25. The light guide is used to irradiate the inside of a body with a light introduced from the above-described light source.

Incidentally, as the endoscope 24, either a hard endoscope or a soft endoscope can be adopted.

As shown in FIG. 1, the HMD 30 is attached to the heat part of the operator OP. The HMD 30 is provided with a left-right pair of display sections 32 at positions corresponding to the respective eyes of the operator OP, facing the front of the face of the operator OP. The display sections 32 are used to display, for example, a color image in a three dimensional format. Incidentally, the display sections 32 are not limited to such an example, and may be one that displays a monochrome image in a two dimensional format.

The entire HMD 30 follows the movement of the head part of the operator OP. That is, as shown by arrows in FIG. 1, in a view from the operator OP side, the HMD 30 is allowed to rotate (right turning) in the right direction (clockwise) with the neck as the central axial line, rotate (left turning) in the left direction (counterclockwise) with the neck as the central axial line, rotate (bending or stretching) in the perpendicular direction to the neck, incline (right side bending) in the right direction with respect to the neck, and incline (left side bending) in the left direction with respect to the neck.

Further, the HMD 30 is provided with a sensor section 3 including the gyro sensor 36 and the geomagnetic sensors 34 (see FIG. 2) for detecting the above-described rotating, side bending, bending, and stretching of the HMD 30. Detected outputs from the gyro sensor 36 and the geomagnetic sensors 34 are provided to the later-described control section 40. Incidentally, acceleration sensors may be used instead of the geomagnetic sensors 34.

The holding arm unit 10 is supported by a mount (not shown) adjacent to an operating table separated from the operator OP, through the bracket (not shown) of a vane motor unit 16. As shown in FIG. 1, the holding arm unit 10 is configured, including a chassis for movably supporting a vane motor 20 that rotatably supports the endoscope 24, a pneumatic cylinder 18 that is fixed to the chassis to make the endoscope 24 and the vane motor 20 close to the patient or distant from the patient, the vane motor unit 16 supported through a parallel link mechanism 14 whose one end portion is supported by the above-described chassis, a rotating shaft section for rotating the above-described entire chassis by being rotated through a timing belt pulley connected to the output shaft of the vane motor unit 16 and a timing belt, and a pneumatic cylinder 12 for driving the parallel link mechanism 14, as main elements.

Incidentally, the vane motor unit 16, the vane motor 20, the pneumatic cylinder 18, the pneumatic cylinder 12, and the like are elements of one example of an actuator, and the parallel link mechanism 14, the timing belt pulley the rotating shaft section, and the like are elements of one example of a displacing mechanism.

One end of a link member constructing a part of the parallel link mechanism 14 is connected to the rotating shaft section, and the other end portion of the link member is connected to the chassis. Thus, for example, when the rod of the pneumatic cylinder 12 connected to the parallel link mechanism 14 is in an elongated state, the chassis in FIG. 1 is clockwise rotated with the lower end of the rotating shaft section as the center. On the other hand, when the pneumatic cylinder 12 is in a contracted state, the chassis in FIG. 1 is counterclockwise rotated with the lower end of the rotating shaft section as the rotation center. That is, as described later, the image capturing section 25 of the endoscope 24 is arranged to be movable in a direction corresponding to the rotation (bending, stretching) of the head part in the perpendicular direction to the neck of the operator OP at the HMD 30, with the rotation center point GP as the center. The rotation center point GP is on a line common with a later-described rotation axis line G of the rotating shaft section, and is located in the vicinity of the body wall of the patient. The rotation axis line G is set such as to be parallel with Lx coordinate axis of the orthogonal coordinate system in FIG. 1 for the holding arm unit 10. Lx coordinate axis is set in a direction perpendicular to the body wall of the patient. Coordinate axis Lz is set perpendicular to Lx coordinate axis.

The pneumatic cylinder 18 is supported by the chassis such that the rod thereof is substantially parallel to the central axis line of the endoscope 24. When the rod of the pneumatic cylinder 18 is elongated, the image capturing section 25 of the endoscope 24and the vane motor 20 in FIG. 1 move with the entire chassis with these attached, in a direction separating from the patient. On the other hand, when the rod of the pneumatic cylinder 18 is contracted, the image capturing section 25 of the endoscope 24 and the vane motor 20 in FIG. 1 are moved with the chassis with these attached, in a direction approaching to the patient.

At positions on the rotating shaft section arranged in parallel with the vane motor unit 16, the positions being separated from each other with a certain interval along the central axis line of the rotating shaft section, one ends of link members constructing the parallel link mechanism 14 are respectively connected. The rotating shaft section is supported by the vane motor unit 16 rotatably around the rotation axis line G. Thus, when the vane motor unit 16 is made in an operation state, the image capturing section 25 and the vane motor 20 can rotate around the rotation axis line G. That is, as described later, the image capturing section 25 is made movable in a direction corresponding to the rotation of the head part of the operator OP at the HMD 30 around the neck.

The part of the endoscope 24, the part being in the vicinity of the operating section, is rotatably supported by the vane motor 20. Thus, the image capturing section 25 of the endoscope 24 can rotate (roll) by a certain angle around the rotation axis line G of the vane motor 20. That is, as described later, the image capturing section 25 of the endoscope 24 is moved in a direction corresponding to the side bending of the operator OP at the HMD 30.

Further, in the one example of the endoscopic operating system 1 according to the present embodiment, as shown in FIGS. 1 and 2, the endoscopic operating system 1 is provided with the control section 40 for performing action control of the holding arm unit 10 and an endoscope control system 60.

As shown in FIG. 2, the endoscope control system 60 is configured, including an endoscope control unit 64 for performing operation control of a zooming mechanism section (not shown) of the endoscope 24 and the light source, based on a group of instruction signals from the operating section 62, and an image processing PC 66 for performing a certain image process, based on image capturing data DD obtained from the solid-state image sensing device of the endoscope 24 via the endoscope control unit 64. Incidentally, the zooming mechanism section can be implemented by general means capable of performing zooming in and zooming out of an image captured by the image capturing section 25.

The image processing PC 66 performs a certain image process, based on image capturing data DD, forms image data ID, and provides image data ID to the control section 40 and the HMD 30. Thus, an image based on the image data ID from the image processing PC 66 is displayed on the display sections 32 of the HMD 30 in a three dimensional format.

Then, as shown in FIG. 2, to the control section 40, transmitted are a group of signals GS representing angular velocity vectors in the above-described respective directions of the head part of the operator OP output from the gyro sensor 36 of the HMD 30, a group of signals EM representing inclination angles in the above-described respective directions of the head part of the operator OP output from the respective geomagnetic sensors 34, an instruction signal Cf representing instruction to stop the action of the holding arm unit 10 from an ON-OFF switching foot switch 50, and an instruction signal Cz1 representing an instruction to increase the zoom amount of the endoscope 24 by a certain amount or an instruction signal Cz2 representing an instruction to decrease the zoom amount of the endoscope 24 by a certain amount, the instruction Cz1 or Cz2 being output from the upper body gyro sensor 37.

The control section 40 is provided with a storage section 40M for storing program data on the vane motor unit 16, the vane motor 20, and air pressure control of the pneumatic cylinder 12 and the pneumatic cylinder 18, image data ID from the image processing PC 66, data representing a computation result by a velocity control computing section 48, the group of signals EM representing the inclination angles output from the geomagnetic sensors 34, and the like.

The control section 40 includes a communicating section 42 for bi-directional transmitting and receiving of control data CD to and from the communicating section 54 of a valve unit controller 56. Based on control data CD from the control section 40, the valve unit controller 56 forms control signals DM1, DM2, DC1, and DC2 to control the vane motor unit 16, the vane motor 20, the pneumatic cylinder 12, and the pneumatic cylinder 18 of the above-described holding arm unit 10, and transmits these signals to a valve unit 58. Based on the control signals DM1, DM2, DC1, and DC2, the valve unit 58 controls respective valves, and supplies operating air from an air supply source to the vane motor unit 16, the vane motor 20, the pneumatic cylinder 12, and the pneumatic cylinder 18 of the holding arm unit 10.

Incidentally, although in the above-described example the valve unit controller 56 is provided, the invention is not limited to this example. For example, instead of using the valve unit controller 56, the control section 40 and the valve unit 58 maybe directly wired with each other so that the holding arm unit 10 is controlled by the control section 40.

The control section 40 controls the insertion amount and the velocity of the inserting portion of the endoscope 24 into the body of the patient, and controls the holding arm unit 10 to make the holding arm unit 10 act in order perform attitude control of the image capturing section 25 of the endoscope 24.

As shown in FIG. 1, the velocity control computing section 48 of the control section 40 includes the computing unit 45, the transforming unit 46, and the drive control unit 47.

Herein, the computing unit 45 computes the angular velocity and the translation velocity from a movement detected by the sensor section 3.

The transforming unit 46 transforms the angular velocity and the translation velocity into a target angular velocity vector $\omega_{ref}$ and a target translation velocity vector $v_{ref}$, taking into account the image capturing angle $\theta$ of the image capturing section 25 formed by the joint section 26, further transforms into a velocity target value $P_{ref}$ of the displacing mechanism, using these, and thereby obtains a position target value $Q_{ref}$. Incidentally, the velocity target value $P_{ref}$ can be obtained from the target angular velocity vector $\omega_{ref}$ and the target translation velocity vector $v_{ref}$, for example, using the Jacobian matrix of the holding arm unit 10. The position target value $Q_{ref}$ can be obtained by computation of integrating the velocity target value $P_{ref}$ and then computation of inverse kinematics. Incidentally, the integration computation and the inverse kinematics computation in obtaining the velocity target value $P_{ref}$ can be performed by a general computation method for robotics.

The drive control unit 47 makes the actuator drive, according to the position target value $Q_{ref}$, and controls the holding arm unit 10.

Transformation into the target angular velocity vector $\omega_{ref}$ and the target translation velocity vector $v_{ref}$ by these respective units, further, transformation into the velocity target value $P_{ref}$, computation of the position target value $Q_{ref}$, and the like, which are performed using the above, are performed in the following manner.

That is, the velocity control computing section 48 sets, by the respective units thereof, the velocity target value $P_{ref}$ of the image capturing section 25 of the endoscope 24 and further sets the position target value $Q_{ref}$, based on the instruction signal Cz1 from the upper body gyro sensor 37of the HMD 30 representing an instruction to increase the insertion amount of the inserting portion of the endoscope 24 by a certain amount into the body, or an instruction signal Cz2 representing an instruction to decrease the insertion amount of the inserting portion of the endoscope 24 by a certain amount, and the group of signals GS from the gyro sensor 36 of the HMD 30 representing the angle velocity vectors of the above-described respective directions of the head part of the operator OP. In order that the image capturing section 25 of the endoscope 24 follows the position target value $Q_{ref}$, based on the position target value $Q_{ref}$, a control data forming section 44 forms control data CD and transmits the control data CD to the communicating section 42 to make the pneumatic cylinder 18 and the vane motor unit 16 of the holding arm unit 10 operate

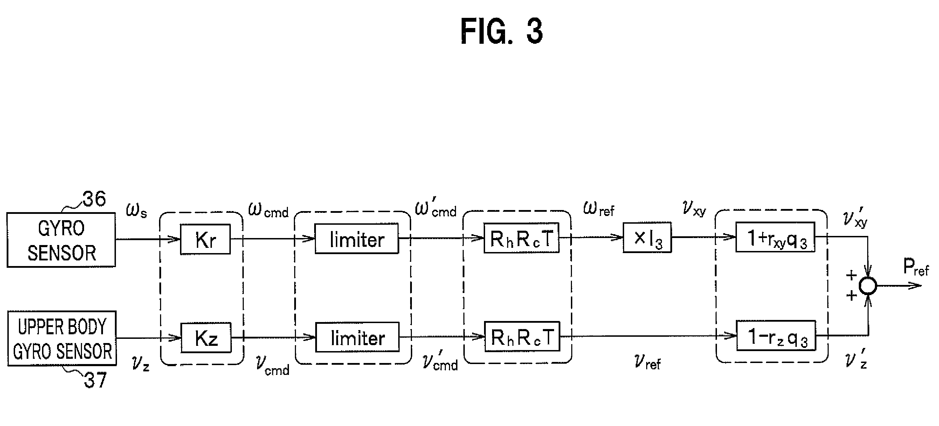

Concretely, the velocity control computing section 48 performs computation by a later described computation expression, according to respective computation steps shown in FIG. 3.

First, the computing unit 45 of the velocity control computing section 48 computes an angular velocity instruction vector $\omega_{cmd}$ by Expression (7), based on the group of signals GS representing the angular velocities from the gyro sensor 36.

$$\omega_{cmd} = K_r \cdot \omega_s \ \ldots (7)$$

Herein, $K_r$ represents velocity gain represented by a later-described matrix, and $\omega_s$ is an angular velocity vector of the head part obtained from the gyro sensor 36 represented by Expression (8). Herein, as the coordinate system, the coordinate system that is set for the head part is used. The central axis of the neck of the operator OP shown in FIG. 1 is defined as y axis, the leftward-rightward direction of the operator OP is defined as x axis, and the forward-backward direction of the operator OP is defined as z axis.

$$\omega_s = (\omega_{sx}, \ \omega_{sy}, \ \omega_{sz})^t \ldots (8)$$

Incidentally, in Expression (8), $\omega_{sx}$, $\omega_{sy}$, and $\omega_{sz}$ respectively represent the coordinates of x axis, y axis, and z axis of the coordinate system that is set for the heat part of the operator OP. Further, t represents the matrix is a transposed matrix.

Further, it is possible to set the sensitivity of movement by multiplying the angular velocities by a constant $K_r$ expressed by Expression (9), matching the taste of a user. The constant $K_r$ can be set to a different value to individual direction. Incidentally, $K_r$ may be a function.

The computing unit 45 limits the angular velocity instruction vector $\omega_{cmd}$ computed by Expression (7) to a certain limit value $\omega_{lim}$ by a limiter, and sets the angular velocity instruction vector $\omega_{cmd}$ to an angular velocity instruction vector $\omega'_{cmd}$. That is, if the angular velocity instruction vector $\omega_{cmd}$ computed by Expression (7) exceeds the limit value $\omega_{lim}$, the angular velocity instruction vector $\omega_{cmd}$ is set to the angular velocity instruction vector $\omega'_{cmd}$ by the limit value $\omega_{lim}$. On the other hand, if the angular velocity instruction vector $\omega_{cmd}$ computed by Expression (7) is smaller or equal to the limit value $\omega_{lim}$, the angular velocity instruction vector $\omega_{cmd}$ is set as the angular velocity instruction vector $\omega'_{cmd}$. This is performed in order to prevent the holding arm unit 10 from acting at an excessive velocity so that the image capturing section 25 does not damage internal organs. Incidentally, the data of the value of the angular velocity instruction vector $\omega'_{cmd}$ is stored in a storage section 40M. In later-described Expression (10), the angular velocity instruction vector $\omega'_{cmd}$ limited by the limit value $\omega_{lim}$ is used.

Subsequently, the transforming unit 46 of the velocity control computing section 48 transforms, according to Expression (10), the angular velocity instruction vector $\omega'_{cmd}$ into local coordinates (Lx, Ly, Lz) (see Fig.

1) of a holding arm by a transformation matrix T, and performs multiplication by a matrix $R_h$ to thereby obtain the angular velocity instruction vector $\omega_{ref}$ of an orthogonal coordinate system (Cx, Cy, Cz) at the tip end portion of the endoscope 24 (Expression (10)). Coordinate axis Cz of the orthogonal coordinate system is taken along the central axis line G of the inserting portion of the endoscope 24, i.e., along the forward direction or the backward direction of the image capturing section 25 of the endoscope 24. Incidentally, the transformation matrix T is a transformation matrix for transformation from a coordinate system being set for the sensor section 3 into a coordinate system being set for the holding arm unit 10, and is always constant. Incidentally, the transformation matrix T is represented by Expression (11). E in Expression (11) represents a rotation matrix, and k and j respectively represent rotation around z axis and rotation around y axis. Accordingly, for example, $E^{k-\pi/2}$ means a matrix for rotation around z axis by -90°.

$$\omega_{ref} = R_h R_e T \cdot \omega'_{cmd} \; ...(10)$$

$$T = E^{k-\pi/2} E^{j-\pi/2} \; ...(11)$$

Matrix $R_h$ in Expression (10) is a matrix representing the attitude of the holding arm unit 10, and can be obtained by computation of forward kinematics in the Expression (12) below from a displacement q by the displacing mechanism. Incidentally, E in Expression (12) represents a rotation matrix; i, j, and k respectively represent rotations around x axis, y axis, and z axis; and q1, q2, and q4 respectively represent displacements by the displacing mechanism (see FIG. 1).

$$R_h = E^{iq1} E^{jq2} E^{kq4} \; ...(12)$$

Herein, in the endoscopic operating system 1 in the present embodiment, matrix $R_c$ is introduced in Expression (10) in order to enable intuitive operation, regardless of the image capturing angle θ of the endoscope 24.

$R_c$ is a matrix representing the image capturing direction of the image capturing section 25. $R_c$ is an identity matrix for a straight scope for example, and is expressed by Expression (13), making the image capturing angle as θ, if the image capturing angle is downward for example. For example, for a 30° oblique scope, $R_c$ can be represented with θ=π/6. Herein, j in Expression (13) is the same as described above.

$$R_c = E^{j\theta} \; ...(13)$$

In the present embodiment, by introducing matrix $R_c$ in Expression (10), the upward-downward and leftward-rightward directions in the screen of the display sections 32 of the HMD 30 and upward-downward and leftward-rightward directions of the head part of the operator OP always agree with each other, regardless of the image capturing angle of the image capturing section 25. That is, the coordinate system that is set for the head part at the HMD 30 and the coordinate system that is set for the image capturing direction of the image capturing section 25 always agree with each other. Accordingly, regardless of the image capturing angle of the image capturing section 25, an image displayed on the display sections 32 of the HMD 30 follows the movement of the head part of the operator OP, which always enables intuitive operation.

Incidentally, in the above-described example, the angular velocity instruction vector $\omega'_{cmd}$ is transformed into the local coordinates (Lx, Ly, Lz) of the holding arm unit 10 by the transformation matrix T and is further multiplied by matrix $R_h$ and matrix $R_c$ to obtain the angular velocity instruction vector $\omega_{ref}$ in the orthogonal coordinate system (Cx, Cy, Cz) at the tip end portion of the endoscope 24, however, the invention is not limited to this example. It is also possible to omit transformation from the local coordinates (Lx, Ly, Lz) of the holding arm unit 10 to the orthogonal coordinate system (Cx, Cy, Cz) at the tip end portion of the endoscope 24. For example, in a case of viewing an image displayed on the display sections 32 of the HMD 30 as an external CRT image. In enabling superimposing of this CRT image and a CT image, it is possible to omit transformation from the local coordinated (Lx, Ly, Lz) of the holding arm unit 10 to the orthogonal coordinate system (Cx, Cy, Cz) at the tip end portion of the endoscope 24.

Subsequently, according to Expression (14), the transforming unit 46 transforms the angular velocity instruction vector $\omega_{ref}$ into a target translation velocity vector $v_{xy}$ at the tip end portion (the image capturing section 25) of the endoscope 24. In more detail, the angular velocity instruction vector $\omega_{ref}$ is transformed into an angular velocity instruction vector $v_{xy}$ having components in the upward-downward direction and leftward-rightward direction with respect to the target velocity at the tip end of the endoscope 24 in the orthogonal coordinate system (Cx, Cy, Cz) by taking the cross product with a vector $l_3$ from rotation center point GP of the holding arm unit 10 to the tip end of the endoscope 24.

$$v_{xy} = \omega_{ref} \times l_3 \; ...(14)$$

Further subsequently, the transforming unit 46 performs computation on the target translation velocity vector $v_{xy}$ for adjustment by Expression (15) in order to make the velocity of the image capturing section 25 changeable corresponding to the insertion amount of the image capturing section 25 of the endoscope 24 into the body. Thus, when the insertion amount of the image capturing section 25 of the endoscope 24 in the direction of movement into the body increases, the target translation velocity vector $v'_{xy}$ of the image capturing section 25 of the endoscope 24 becomes large. On the other hand, when the insertion amount of the image capturing section 25 of the endoscope 24 decreases, i.e., when the image capturing section 25 of the endoscope 24 is pulled off from the inside of the body, the target translation velocity vector $v'_{xy}$ of the image capturing section 25 of the endoscope 24 becomes small.

$$v'_{xy} = (1 + r_{xy}\, q_3)\, v_{xy} \ ...(15)$$

By multiplying the respective values of $v_{xy}$ by a factor $r_{xy}$ dependent on the $q_3$ (see FIG. 1) representing the insertion amount of the tip end of the endoscope 24, by Expression (15), the degree of dependence of the movement amount on the screen on the degree of insertion is adjusted. Thus, the movement amount of the visual field by rotation of the head can be adjusted. For example, it is possible to make the movement amount on the screen of an object of viewing at the time when the head is rotated be substantially constant, regardless of the zooming position. Accordingly, the intuitiveness of operation is improved.

Herein, $r_{xy}$ is a constant and is set in a range that the positive and negative of $v_{xy}$ are not reversed. It is assumed herein that $q_3$ is positive for the direction in which the endoscope 24 is inserted from the midpoint and negative for the direction in which the endoscope 24 is pulled off. The center of the movable range of $q_3$ in FIG. 1 is defined as the midpoint and the midpoint is set to zero. Incidentally $r_{xy}$ may be a function.

On the other hand, according to Expression (16), the computing unit 45 of the velocity control computing section 48 computes the translation velocity (translation velocity vector $v_{cmd}$) along the Cz coordinate axis (see FIG. 1) of the image capturing section 25 of the endoscope 24, based on the velocity $v_z$ in the forward-backward direction of the head part obtained from the upper body gyro sensor 37.

Incidentally, in Expression (16), $K_z$ represents gain having been set by a user such as the operator OP, and t means the same as described above.

$$v_{cmd} = (0,\, 0,\, K_z v_z)^{\ t} \ ...(16)$$

Further, in the computing unit 45, a target velocity instruction vector $v_{cmd}$ computed by Expression (16) is limited to a certain limit value $v_{lim}$ by a limiter, and is set to a target velocity instruction vector $v'_{cmd}$. In more detail, if the target velocity instruction vector $v_{cmd}$ computed by Expression (16) exceeds the limit value $v_{lim}$, the target velocity instruction vector $v_{cmd}$ is set by the limit value $v_{lim}$ to the target velocity instruction vector $v'_{cmd}$. On the other hand, if the target velocity instruction vector $v_{cmd}$ computed by Expression (16) is lower than or equal to the limit value $v_{lim}$, the target velocity instruction vector $v_{cmd}$ is set as this target velocity instruction vector $v'_{cmd}$. This setting is performed to prevent the holding arm unit 10 from acting at an excessive velocity. By restricting the operation of the holding arm unit 10 to prevent operation at an excessive velocity, it is possible to improve the safety so as to prevent the endoscope 24 from hitting against an internal organ and damaging it. In the later-described Expression (17), the target velocity instruction vector $v'_{cmd}$ limited by the limit value $v_{lim}$ is used.

Subsequently, the transforming unit 46 transforms the obtained target velocity instruction vector $v'_{cmd}$ to the target translation velocity vector $v_{ref}$ of the image capturing section 25 of the endoscope 24, according to Expression (17). Thus, it is possible to make the forward-backward movement of the head and the forward-backward movement of the endoscope 24 agree with each other. Herein, matrix $R_h$, matrix $R_c$, and transformation matrix T mean the same as the above described.

$$v_{ref} = R_h\, R_c\, T \cdot v'_{cmd} \ ...(17)$$

Matrix $R_c$ is also introduced to Expression (17). Consequently, the upward-downward direction and the leftward-rightward direction in the screen on the display sections 32 of the HMD 30 and the upward-downward direction and the leftward-rightward direction of the head part of the operator OP always agree with each other, regardless of the image capturing angle of the image capturing section 25. That is, the coordinate system that is set for the head part at the HMD 30 and the coordinate system that is set in the image capturing direction of the image capturing section 25 always agree with each other. Accordingly, an image displayed on the display sections 32 of the HMD 30 follows the movement of the head part of the operator OP, regardless of the image capturing angle of the image capturing section 25, which always enables intuitive operation.

Then, further, in order to adjust the velocity of the image capturing section 25 so that the velocity becomes changeable, corresponding to the insertion amount of the image capturing section 25 of the endoscope 24 into the body, the transforming unit 46 computes a target translation velocity vector $v'_z$ by Expression (18), using the target translation velocity vector $v_{ref}$ obtained by Expression (17). Incidentally, in Expression (18), $r_z$ may be either a constant or a function. Herein, $q_3$ means the same as described above.

$$v'_z = (1 - r_z q_3)\, v_{ref} \ ...(18)$$

The upward-downward and leftward-rightward action (movement of the rotations $q_1$, $q_2$ of the holding arm unit 10) (see FIG. 1) is magnified in zooming in (in deep insertion) and reduced in zooming out. The forward-backward movement (movement of $q_3$ in insertion of the endoscope 24 of the holding arm unit 10) acts the opposite. Thus, the magnification amount of a viewed object on the screen in zooming can be made substantially constant, regardless of the zoom position. Further, as the zoom movement amount in deep insertion becomes small, unexpected contact between the endoscope 24 and an internal organ can be avoided.

Then, using the target translation velocity vector $v'_{xy}$ obtained by Expression (15) and the target translation velocity vector $v'_z$ obtained by Expression (18), the transforming unit 46 adds the velocity components in the upward-downward direction and in the forward-backward direction, according to Expression (19) to thereby obtain the final velocity target value $P_{ref}$ at the tip end (image capturing section) of the endoscope.

$$P_{ref} = v'_{xy} + v'_z \ldots (19)$$

Further, subsequently, as described above, the transforming unit 46 performs integration computation on this velocity target value $P_{ref}$ in a general manner and obtains a position target value $Q_{ref}$ by computation of inverse kinematics.

Then, the drive control unit 47 drives the above-described actuator, according to the position target value $Q_{ref}$ obtained in such a manner.

Incidentally, in the above-described example, the roll component (action of inclining the neck) of the rotation velocity of the head part of the operator OP is given from the roll component of the above-described angular velocity instruction vector $\omega'_{cmd}$ directly as the target velocity of the roll $q_4$ of the endoscope, however, the invention is not limited to this example. Further, this action may be made ineffective.

Further, although, in the above description, an instruction of the forward-backward direction is made by a foot switch, the invention is not limited to this manner. As another manner, generation of a forward-backward direction instruction value by an accelerator sensor, an optical flow, measurement of the skin displacement or muscle potential in the vicinity of the glabella may be performed.

Effects obtained by using the ON-OFF switching foot switch 50 include the flowing. When it is desired not to operate the endoscope 24, the head can be freely moved by switching off the ON-OFF switching foot switch 50. Further, for example, in moving the endoscope 24 to the right with the switch ON, even when the own head has reached the right movable limit, the endoscope 24 can be further moved to the right by turning the switch OFF and returning the head to the left first and then tuning the switch ON. Still further, as long as the switch is not turned ON, as the endoscope 24 does not move in association with the head, it is possible to avoid unexpected operation or action.

The above-described endoscopic operating system 1 according to the invention transforms the movement of the operator OP into a target angular velocity vector $\omega_{ref}$ and a target translation velocity vector $v_{ref}$ of the holding arm unit 10, taking into account the image capturing angle $\theta$ of the image capturing section 25 made by the joint section 26; further transforms into a velocity target value $P_{ref}$ of the displacing mechanism, using these; and thereafter obtains a position target value $Q_{ref}$ from this velocity target value $P_{ref}$ to drive the actuator, according to this position target value $Q_{ref}$. Herein, as described above, the spatial coordinates of the head of the operator OP and the spatial coordinates of the image capturing section 25 agree with each other, and the position variation of the image capturing section 25 correspondingly agrees with the variation of the position and the acceleration of the head part of the operator OP. In such a manner, it is possible to perform intuitive operation, regardless of the image capturing angle $\theta$ of the image capturing section 25 of the endoscope 24.

[Endoscope Operation Program]

The endoscopic operating program in the present embodiment is a program to operate the above-described endoscopic operating system 1 in the present embodiment. IN order to operate the endoscopic operating system 1, this program makes a computer function as a computing unit, a transforming unit, and a drive control unit.

The computing unit, the transforming unit, and the drive control unit for this program correspond to the computing unit 45, the transforming unit 46, and the drive control unit 47 in the above description of the endoscopic operating system 1. Accordingly, detailed description is omitted here.

An endoscopic operating program according to the invention may be recorded in a computer readable recording medium (not shown) such as a CD-ROM, a flexible disk, read out from this recording medium by a recording medium driving device (not shown), and installed on a storage unit, not shown, to be executed.

Further, if a computer (client) that functions as the endoscopic operating system 1 is provided with communication means such as a communication network, the endoscopic operating program according to the invention may be stored in another computer (server) connected via the communication network, and arrangement may be made such as to download the endoscopic operating program via the communication network from this computer (server) to execute the endoscopic operating program, or execute the endoscopic operating program according to the invention stored in the server, so as to transform the angular velocity and the translation velocity into the target angular velocity vector and the target translation velocity vector of the holding arm unit 10, taking into account the image capturing angle of the image capturing section 25 changed by the joint section 26, further transform into the velocity target value of the displacing mechanism, using these, and obtain the position target value from this velocity target value to thereby drive the actuator. In this case, a result of numerical analysis may be stored in a storage unit (not shown) provided in the server.

Description of Reference Symbols

| 1: | endoscopic operating system |
| 3: | sensor section |
| 10: | holding arm unit |
| 25: | image capturing section |
| 26: | joint section |
| 40: | control section |
| 45: | computing unit |
| 46: | transforming unit |
| 47: | drive control unit |

**Claims**

1. An endoscopic operating system, comprising:

    a sensor section for detecting movement of at least one of a head part and an upper body of an operator;
    a control section for driving one or more actuators, corresponding to the movement detected by the sensor section;
    a holding arm unit supported to be reciprocatable and rotatable by the actuator and one or more displacing mechanisms connected to the actuator;
    an image capturing section provided at an arbitrary part of the holding arm unit through a joint section capable of freely change an image capturing angle by the actuator; and
    a display section for displaying an image captured by the image capturing section on a screen,
    wherein the control section includes:

      a computing unit for computing an angular velocity and a translation velocity from the movement detected by the sensor section;
      a transforming unit for transforming the angular velocity and the translation velocity into a target angular velocity vector and a target translation velocity vector of the holding arm unit, taking into account the image capturing angle of the image capturing section by the joint section, and further performing transformation into a velocity target value of the displacing mechanism by using the target angular velocity vector and the target translation velocity vector in order to obtain a position target value from the velocity target value; and
a drive control unit for driving the actuator according to the position target value.

2. The endoscopic operating system according to claim 1,
   wherein spatial coordinates of the sensor section for detecting the angular velocity and the translation velocity of the head part of the operator are spatial coordinates with a central axis of the neck of the operator as y axis, leftward-rightward direction of the operator as x axis, and forward-backward direction of the operator as z axis,
   wherein special coordinates of the image capturing section are spatial coordinates with leftward-rightward direction of the image capturing section as x axis, upward-downward direction of the image capturing section as y axis, and optical axis direction of the image capturing section as z axis, and
   wherein control is performed to make variation of position and acceleration of the head part of the operator and corresponding position variation of the image capturing section are the same, regardless of a bending state of the holding arm unit and the joint section.

3. The endoscopic operating system according to claim 2,
   wherein in performing the control, the image capturing angle of the image capturing section is represented by a matrix, and the matrix is used in coordinate transformation from the variation of the head part of the operator into position variation of the holding arm unit and the joint section.

4. The endoscopic operating system according to any one of claims 1 to 3,
   wherein the transformation unit transforms the angular velocity and the translation velocity into the target angular velocity vector and the target translation velocity vector of the holding arm unit, based on following Expressions (1) and (2).

$$\omega_{ref} = R_h\, R_e\, T \cdot \omega'_{cmd} \,...(1)$$

$$v_{ref} = R_h\, R_c\, T \cdot v'_{cmd} \,...(2)$$

where in Expressions (1) and (2),

$\omega_{ref}$ represents a target angular velocity vector of the holding arm unit,
$v_{ref}$ represents a target translation velocity vector of the holding arm unit, and
$R_h$ represents a matrix representing attitude of the holding arm unit and is obtained by computation of forward kinematics of Expression (3) below from displacement by the displacing mechanism,
$R_c$ represents a matrix representing image capturing angle $\theta$ of the image capturing section and expressed by Expression (4) below,
T represents a transformation matrix for transformation from a coordinate system that is set for the sensor section into a coordinate system that is set for the holding arm unit,
$\omega'_{cmd}$ is obtained by limiting an angular velocity instruction vector $\omega_{cmd}$ of the holding arm unit by a limiting value, the angular velocity instruction vector $\omega_{cmd}$ being expressed by Expression (5) below, and
$v'_{cmd}$ is obtained by limiting a translation velocity instruction vector $v_{cmd}$ of the holding arm unit by a limiting value, the translation velocity instruction vector $v_{cmd}$ being expressed by Expression (6) below.

$$R_h = E^{iq1}\ E^{jq2}\ E^{kq4}\ ...(3)$$

$$R_c = E^{j\theta}\ ...(4)$$

$$\omega_{cmd} = K_r \cdot \omega_s\ ...(5)$$

$$v_{cmd} = (0,\ 0,\ K_z\,v_z)^t\ ...(6),$$

and
where in Expressions (3) to (6),

E represents a rotation matrix,
i, j, and k respectively represent rotations around x, y, and z axes,
q1, q2, and q4 represent respective displacements by the displacing mechanism,
$\theta$ represents the image capturing angle of the image capturing section,
$K_r$ represents a factor matrix representing a velocity gain,
$\omega_s$ represents a three dimensional angular velocity vector detected by the sensor section,

$K_z$ represents a gain that is set by a user,
$v_z$ represents a velocity in head part forward-backward direction, and
t represents that the matrix is a transposed matrix.

5. An endoscopic operating program for operating the endoscopic operating system according to claim 1, wherein the program causes a computer to serve as:

a computing unit for computing an angular velocity and a translation velocity from a movement detected by the sensor section;
a transforming unit for transforming the angular velocity and the translation velocity into a target angular velocity vector and a target translation velocity vector of the holding arm unit, taking into account image capturing angle of the image capturing section by the joint section, and further performing transformation into a velocity target value of the displacing mechanism by using the target angular velocity vector and the target translation velocity vector in order to obtain a position target value from the velocity target value; and
a drive control unit for driving the actuator, according to the position target value.

## FIG. 1

# FIG. 2

EP 2 979 605 A1

# FIG. 3

# FIG. 4

132

132

110

124

IMAGE CAPTURING ANGLE
$\theta = 0°$

OP

EP 2 979 605 A1

FIG. 5

## INTERNATIONAL SEARCH REPORT

| International application No. |
| --- |
| PCT/JP2013/059725 |

**A.  CLASSIFICATION OF SUBJECT MATTER**
*A61B1/00*(2006.01)i

According to International Patent Classification (IPC) or to both national classification and IPC

**B.  FIELDS SEARCHED**

Minimum documentation searched (classification system followed by classification symbols)
A61B1/00

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
| --- | --- | --- |
| A | JP 8-117238 A  (Olympus Optical Co., Ltd.), 14 May 1996 (14.05.1996), fig. 13, 15 & US 6120433 A | 1-5 |
| A | JP 8-196541 A  (Olympus Optical Co., Ltd.), 06 August 1996 (06.08.1996), fig. 6 to 8, 13, 14 (Family: none) | 1-5 |
| A | JP 9-66056 A  (Olympus Optical Co., Ltd.), 11 March 1997 (11.03.1997), abstract; fig. 1 (Family: none) | 1-5 |

| ☒    Further documents are listed in the continuation of Box C. | ☐    See patent family annex. |
| --- | --- |

| * | Special categories of cited documents: | "T" | later document published after  the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 May, 2013 (10.05.13) | 21 May, 2013 (21.05.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2013/059725

C (Continuation).　DOCUMENTS CONSIDERED TO BE RELEVANT

| Category* | Citation of document, with indication, where appropriate, of the relevant passages | Relevant to claim No. |
|---|---|---|
| A | JP 9-28663 A  (Olympus Optical Co., Ltd.), 04 February 1997 (04.02.1997), paragraphs [0180] to [0184] & US 5836869 A | 1-5 |
| A | JP 2009-285099 A  (Tokyo Institute of Technology), 10 December 2009 (10.12.2009), abstract (Family: none) | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10309258 A


**Non-patent literature cited in the description**

- Naviot. Iryo-yo Naishikyo Haji Souchi. Hitachi Hybrid Network Co., Ltd,